# EUROPEAN PATENT SPECIFICATION

(11) **EP 3 603 609 B1**
(45) Date of publication and mention of the grant of the patent: **13.04.2022**
(21) Application number: 19188672.0
(22) Date of filing: 26.07.2019
(51) Int. Cl.: A61K 8/06, A61K 8/34, A61K 8/87, A61Q 1/02, A61Q 1/06, A61Q 1/10, A61Q 17/04, A61Q 19/00

(54) **COSMETIC EMULSION COMPOSITION**
KOSMETISCHE EMULSIONZUSAMMENSETZUNG
COMPOSITION ÉMULSION COSMÉTIQUE

(30) Priority: 31.07.2018 JP 2018143331
(43) Date of publication of application: 05.02.2020
(73) Proprietor: ADEKA CORPORATION, Arakawa-ku Tokyo 116-8554 (JP)
(72) Inventor: YASUTANI, Akihito, Tokyo, 116-8554 (JP); NAKAJIMA, Kazuya, Tokyo, 116-8554 (JP)
(74) Representative: Beck Greener LLP

(56) References cited:
- EP-A1- 2 565 233
- EP-A1- 2 796 126
- EP-A1- 3 111 918
- JP-A- 2015 086 159
- JP-A- 2018 016 547

## Description

### Technical Field

The present invention relates to a cosmetic emulsion composition that exhibits excellent usability, excellent use sensation, and excellent transparency.

### Background Art

An emulsion composition, i.e., a water-in-oil emulsion composition or an oil-in-water emulsion composition, is commonly used in skin care cosmetics and make-up cosmetics in order to improve usability, i.e., spreadability and smoothness, and improve use sensation when applied, i.e., moistness and softness. Users are also demanding high transparency from such emulsion compositions from the standpoint of enabling the addition of functionality through adjustment of the image or appearance imparted to the user.

Patent Literature 1 describes a cosmetic containing a volatile oil fraction, a hydrophobic powder, and an amphiphilic associative thickener as being a water-in-oil emulsion base make-up cosmetic that exhibits an excellent sense of transparency, a use sensation of moistness without stickiness, and excellent stability. Patent Literature 2 describes a water-in-oil emulsion cosmetic that contains a volatile oil agent, a silicone surfactant, a hydrophobic powder, a partially crosslinked organopolysiloxane polymer, and a hydrophobically modified polyether urethane. This is described as being a cosmetic having the qualities of being resistant to stickiness, providing a uniform cosmetic film, being resistant to collapse into pores both during application and with elapsed time, and not exhibiting a loss of finish when makeup is applied.

In addition, attention has been focused in recent years on glyceryl ether compounds, e.g., 2-ethylhexyl glyceryl ether, n-hexyl glyceryl ether, and so forth, as substitutes for parabens, which have been used in cosmetic compositions as antimicrobial preservatives but which have become disfavored because they have a very strong skin irritation activity. Glyceryl ether compounds have entered into use in numerous applications in recent years because they exhibit an antimicrobial activity, exhibit a lower skin irritancy than the parabens, and are highly safe to humans (for example, Patent Literatures 3 and 4).

### Citation List

[Patent Literature 1] Japanese Patent Application Publication No. 2010-111634
[Patent Literature 2] Japanese Patent Application Publication No. 2017-186322
[Patent Literature 3] Japanese Patent Application Publication No. 2015-086159
[Patent Literature 4] Japanese Patent Application Publication No. 2011-057647

### SUMMARY OF THE INVENTION

### TECHNICAL PROBLEM

As noted above, various emulsion compositions have been disclosed for the purpose of improving the usability and use sensation of cosmetics; however, these are still not cosmetic emulsion compositions that satisfy all of the various properties, i.e., usability, use sensation, and transparency of appearance, at levels demanded by users, and further improvement is required.

### SOLUTION OF THE PROBLEM

The present inventors then achieved the present invention as a result of intensive investigations. That is, the present invention was achieved based on the discovery that a cosmetic emulsion composition having excellent transparency and dramatically improved usability and use sensation can be obtained by the incorporation therein of (A) a component containing a monoalkyl glyceryl ether having a C₆₋₈ alkyl group and (B) a component containing an associative urethane.

### ADVANTAGOUS EFFECT OF INVENTION

The present invention can thus provide a cosmetic emulsion composition that satisfies all of the various properties, i.e., usability, use sensation, and transparency of appearance, at levels demanded by users.

### DESCRIPTION OF EMBODIMENTS

The present invention relates to a cosmetic emulsion composition that exhibits excellent usability, excellent use sensation, and excellent transparency. In specific terms this is a cosmetic emulsion composition that comprises (A) a component containing a monoalkyl glyceryl ether having a C₆₋₈ alkyl group and (B) a component containing an associative urethane.

The monoalkyl glyceryl ether having a C₆₋₈ alkyl group can be exemplified by monoalkyl glyceryl ethers having a C₆₋₈ straight-chain alkyl group, or having a C₆₋₈ branched alkyl group, or having a C₆₋₈ cycloalkyl group. The C₆₋₈ alkyl group can be specifically exemplified by the hexyl group, secondary-hexyl group, heptyl group, secondary heptyl group, octyl group, 2-ethylhexyl group, secondary-octyl group, cyclohexyl group, methylcyclohexyl group, dimethylcyclohexyl group, cycloheptyl group, and methylcycloheptyl group. When the number of carbon atoms is less than 6, desirable usability and use sensation may then not be obtained, and less than 6 carbon atoms is also unfavorable due to the reduction in the antimicrobial activity. When, on the other hand, the number of carbon atoms is more than 8, the solubility in water is then poor and the transparency in the appearance of the cosmetic is poor. More than 8 carbon atoms is also unfavorable because this can be strongly irritating for the skin. A single monoalkyl glyceryl ether having the indicated straight-chain alkyl group, branched alkyl group, or cycloalkyl group may be used or two or more may be used. A dramatic improvement in the usability and use sensation of the cosmetic emulsion composition can be brought about through the co-use of component (A) with component (B).

Among the compounds described above, component (A) is, from the standpoint of bringing about a dramatic improvement in the usability and use sensation of the cosmetic emulsion composition through co-use with component (B), preferably a monoalkyl glyceryl ether having a C₆₋₈ straight-chain alkyl group or a C₆₋₈ cycloalkyl group and is more preferably a monoalkyl glyceryl ether having a C₆ straight-chain alkyl group or a C₆ cycloalkyl group. Considered in terms of facilitating the appearance of the effects of the present invention while also supporting the appearance of an antimicrobial preservative activity, component (A) preferably contains a monoalkyl glyceryl ether having a C₆ cycloalkyl group.

The amount of incorporation of component (A) is not particularly limited; however, from the standpoint of bringing about additional improvements in the transparency of the cosmetic emulsion composition, and considered with reference to the overall amount of the cosmetic emulsion composition, its content is preferably 0.01 to 10 mass%, more preferably 0.1 to 5.0 mass%, still more preferably 0.5 to 4.0 mass%, even more preferably 1.0 to 3.0 mass%, and particularly preferably 1.2 to 2.5 mass%.

Component (A) is known to have an antimicrobial preservative activity, and as a result the cosmetic emulsion composition according to the present invention can be expected to have a high antimicrobial preservative activity. In terms of providing the cosmetic emulsion composition with a high antimicrobial preservative activity, the component (A) content is preferably 0.5 to 4.0 mass% and more preferably 1.0 to 3.0 mass%, in each case with reference to the overall amount of the cosmetic emulsion composition.

The associative urethane that is component (B) in the present invention is a urethane-type copolymer that has a hydrophilic group portion for the backbone and a hydrophobic moiety in terminal position. Associative urethanes are thought to exhibit a thickening action through association between the hydrophobic moieties of the copolymer in an aqueous medium while the hydrophilic portion forms a loop shape or bridge shape. There are no particular limitations on this associative urethane, but examples are the compounds described in, for example, Japanese Patent Application Publication No. H06-340805 and Japanese Translation of PCT Application No. 2011-520003, which corresponds to WO 2009/135859.

From the standpoint of further increasing the usability and use sensation of the cosmetic emulsion composition through co-use with component (A), component (B) preferably contains the hydrophobically modified polyether urethane represented by the following general formula (I).

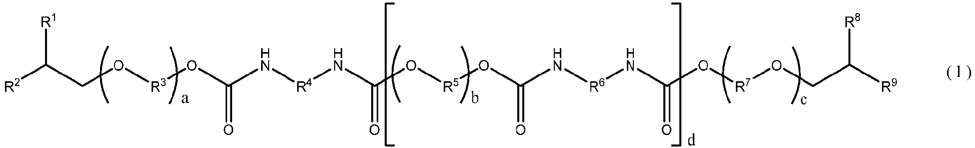

In the formula, R¹, R², R⁸, and R⁹ each independently represent a C₁₋₂₀ hydrocarbon group; R³, R⁵, and R⁷ each independently represent a C₂₋₄ divalent hydrocarbon group; R⁴ and R⁶ each independently represent a C₃₋₁₆ divalent hydrocarbon group; a and c each independently represent a number from 10 to 100; b represents a number from 100 to 500; and d represents a number from 0 to 10. Such a hydrophobically modified polyether urethane can be exemplified by the compounds described in WO 2018/043374.

R¹, R², R⁸, and R⁹ in general formula (I) each independently represent a C₁₋₂₀ hydrocarbon group. This hydrocarbon group can be exemplified by saturated aliphatic hydrocarbon groups such as the methyl group, ethyl group, n-propyl group, t-propyl group, n-butyl group, isobutyl group, s-butyl group, t-butyl group, n-pentyl group, branched-chain pentyl group, secondary-pentyl group, tertiary-pentyl group, n-hexyl group, branched-chain hexyl group, secondary-hexyl group, tertiary-hexyl group, n-heptyl group, branched-chain heptyl group, secondary-heptyl group, tertiary-heptyl group, n-octyl group, 2-ethylhexyl group, branched-chain octyl group, secondary-octyl group, tertiary-octyl group, n-nonyl group, branched-chain nonyl group, secondary-nonyl group, tertiary-nonyl group, n-decyl group, branched-chain decyl group, secondary-decyl group, tertiary-decyl group, n-undecyl group, branched-chain undecyl group, secondary-undecyl group, tertiary-undecyl group, n-dodecyl group, branched-chain dodecyl group, secondary-dodecyl group, tertiary-dodecyl group, n-tridecyl group, branched-chain tridecyl group, secondary-tridecyl group, tertiary-tridecyl group, n-tetradecyl group, branched-chain tetradecyl group, secondary-tetradecyl group, tertiary-tetradecyl group, n-pentadecyl group, branched-chain pentadecyl group, secondary-pentadecyl group, tertiary-pentadecyl group, n-hexadecyl group, branched-chain hexadecyl group, secondary-hexadecyl group, tertiary-hexadecyl group, n-heptadecyl group, branched-chain heptadecyl group, secondary-heptadecyl group, tertiary-heptadecyl group, n-octadecyl group, branched-chain octadecyl group, secondary-octadecyl group, tertiary-octadecyl group, n-nonadecyl group, branched-chain nonadecyl group, secondary nonadecyl group, tertiary-nonadecyl group, n-eicosyl group, branched-chain eicosyl group, secondary-eicosyl group, and tertiary-eicosyl group; unsaturated aliphatic hydrocarbon groups such as the 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group, 1-heptenyl group, 6-heptenyl group, 1-octenyl group, 7-octenyl group, 8-nonenyl group, 1-decenyl group, 9-decenyl group, 10-undecenyl group, 1-dodecenyl group, 4-dodecenyl group, 11-dodecenyl, 12-tridecenyl group, 13-tetradecenyl, 14-pentadecenyl group, 15-hexadecenyl group, 16-heptadecenyl group, 1-octadecenyl group, 17-octadecenyl group, 1-nonadecenyl group, and 1-eicosenyl group;

aromatic hydrocarbon groups such as the phenyl group, tolyl group, xylyl group, cumenyl group, mesityl group, benzyl group, phenethyl group, styryl group, cinnamyl group, benzhydryl group, trityl group, ethylphenyl group, propylphenyl group, butylphenyl group, pentylphenyl group, hexylphenyl group, heptylphenyl group, octylphenyl group, nonylphenyl group, decylphenyl group, undecylphenyl group, dodecylphenyl group, styrenated phenyl group, p-cumylphenyl group, phenylphenyl group, benzylphenyl group, α-naphthyl group, and **β**-naphthyl group; and alicyclic hydrocarbon groups such as the cyclopropyl group, cyclobutyl group, cyclopentyl group, cyclohexyl group, cycloheptyl group, cyclooctyl group, methylcyclopentyl group, methylcyclohexyl group, methylcycloheptyl group, methylcyclooctyl group, 4,4,6,6-tetramethylcyclohexyl group, 1,3-dibutylcyclohexyl group, norbornyl group, bicyclo[2.2.2]octyl group, adamantyl group, 1-cyclobutenyl group, 1-cyclopentenyl group, 3-cyclopentenyl group, 1-cyclohexenyl group, 3-cyclohexenyl group, 3-cycloheptenyl group, 4-cyclooctenyl group, 2-methyl-3-cyclohexenyl group, and 3,4-dimethyl-3-cyclohexenyl group.

R¹, R², R⁸ and R⁹ may be the same as each other or may differ from one another. In order to obtain a hydrophobically modified polyether urethane that readily generates the effects of the present invention, and based on ease of starting material acquisition and ease of production, C₁₋₂₀ saturated aliphatic hydrocarbon groups and C₁₋₂₀ unsaturated aliphatic hydrocarbon groups are preferred among the preceding, C₁₋₂₀ saturated aliphatic hydrocarbon groups are more preferred, C₅₋₁₈ saturated aliphatic hydrocarbon groups are even more preferred, C₈₋₁₄ saturated aliphatic hydrocarbon groups are still more preferred, and C₁₀₋₁₂ saturated aliphatic hydrocarbon groups are most preferred. In specific terms, R¹ and R⁸ are preferably the decyl group and R² and R⁹ are preferably the dodecyl group.

R³, R⁵, and R⁷ in general formula (I) each independently represent a C₂₋₄ divalent hydrocarbon group. Examples of this hydrocarbon group are the ethylene group; propan-1,3-diyl (straight-chain propylene) group; branched chain propylene groups such as the propan-1,2-diyl group and propan-2,2-diyl group; straight-chain butylene groups such as the butan-1,4-diyl group, butan-1,2-diyl group, butan-1,3-diyl group, butan-2,3-diyl group, butan-1,1-diyl group, and butan-2,2-diyl group; and branched-chain butylene groups such as the 2-methylpropan-1,3-diyl group and 2-methylpropan-1,2-diyl group. Among these, C₂₋₄ divalent straight-chain hydrocarbon groups are preferred because they provide compounds with which the effects of the present invention can be readily obtained, and the ethylene group and propan-1,3-diyl (straight-chain propylene) group are more preferred and the ethylene group is still more preferred. The R³ may all be the same group or may be different groups; the R⁵ may also all be the same group or may be different groups; and the R⁷ may also all be the same group or may be different groups.

R⁴ and R⁶ in general formula (I) each independently represent a C₃₋₁₆ divalent hydrocarbon group. Examples of this hydrocarbon group are C₃₋₁₆ divalent aliphatic hydrocarbon groups, C₃₋₁₆ aromatic hydrocarbon groups, and C₃₋₁₆ alicyclic hydrocarbon groups. Any of these hydrocarbon groups having a number of carbon atoms in the range from 3 to 16 may be used, but a group provided by the removal of the two isocyanate groups from a diisocyanate compound is preferred for the corresponding ease of production and ease of starting material acquisition. The diisocyanate compound can be exemplified by aliphatic diisocyanates such as trimethylene diisocyanate, tetramethylene diisocyanate, pentamethylene diisocyanate, hexamethylene diisocyanate (HDI), 2,2-dimethylpentane diisocyanate, octamethylene diisocyanate, 2,2,4-trimethylpentane diisocyanate, nonamethylene diisocyanate, decamethylene diisocyanate, dodecamethylene diisocyanate, isophorone diisocyanate (IPDI), dicyclohexylmethane diisocyanate (hydrogenated MDI), hydrogenated xylylene diisocyanate (hydrogenated XDI), and 2,4,4(or 2,2,4)-trimethylhexamethylene diisocyanate (TMDI); and by aromatic diisocyanates such as tolylene diisocyanate (TDI), diphenylmethane diisocyanate (MDI), toluidine diisocyanate (TODI), xylylene diisocyanate (XDI), and naphthalene diisocyanate (NDI). In specific terms, a C₆ hydrocarbon group is preferred for each of R⁴ and R⁶, and the group provided by removing the two isocyanate groups from hexamethylene diisocyanate, i.e., the hexylene group, is more preferred.

The a and c in general formula (I) each independently represent a number from 10 to 100. 12 to 50 is preferred and 15 to 30 is more preferred, because this facilitates obtaining the effects of the present invention and due to the ease of starting material production or acquisition. a and c are specifically preferably each 20.

b in general formula (I) represents a number from 100 to 500. In order to obtain a hydrophobically modified polyether urethane that facilitates obtaining the effects of the present invention, 120 to 450 is preferred, 150 to 400 is more preferred, 180 to 350 is still more preferred, and 200 to 300 is most preferred. In specific terms b is preferably 240.

d in general formula (I) represents a number from 0 to 10. In order to obtain a hydrophobically modified polyether urethane that facilitates obtaining the effects of the present invention, 0 to 8 is preferred and 0 to 6 is more preferred. A hydrophobically modified polyether urethane in which d is 0 exhibits activity as a gelation promoter through its co-use with a hydrophobically modified polyether urethane in which d is 1 to 10. Consequently, in order to further facilitate obtaining the effects of the present invention, a mixture of a hydrophobically modified polyether urethane in which d is 0 and a hydrophobically modified polyether urethane in which d is 1 to 10 is more preferred, while a mixture of a hydrophobically modified polyether urethane in which d is 0 and a hydrophobically modified polyether urethane in which d is 1 to 8 is still more preferred and a mixture of a hydrophobically modified polyether urethane in which d is 0 and a hydrophobically modified polyether urethane in which d is 1 to 6 is most preferred. More particularly, the effects of the present invention can be maximally expressed with a hydrophobically modified polyether urethane in which (hydrophobically modified polyether urethane in which d is 1 to 10) and (hydrophobically modified polyether urethane in which d is 0) are mixed in a mass ratio therebetween in proportions of 95 : 5 to 85 : 15.

The content of component (B) is not particularly limited, but from the standpoint of further enhancing the usability and use sensation of the cosmetic emulsion composition, it is, with reference to the overall amount of the cosmetic emulsion composition, preferably 0.01 to 5.0 mass%, more preferably 0.1 to 3.0 mass%, and still more preferably 0.2 to 2.0 mass%.

The mass ratio between the contents of component (A) and component (B) in the cosmetic emulsion composition is not particularly limited, but from the standpoint of dramatically enhancing the usability and use sensation of the cosmetic emulsion composition through the co-use of component (A) with component (B), component (B) is, per 1 mass part of component (A), preferably 0.01 to 5 mass parts, more preferably 0.1 to 2 mass parts, still more preferably 0.2 to 1 mass parts, and even more preferably 0.3 to 0.5 mass parts.

The cosmetic emulsion composition according to the present invention may also contain a polyether-modified silicone-type surfactant as a component (C). A polyether-modified silicone-type surfactant is a surfactant composed of a polymer having a silicone chain, which is the main chain, and a polyether chain introduced by modification. The silicone chain may be a straight-chain silicone chain or may be a branched-chain silicone chain. The polyether chain is exemplified by the polyethylene glycol (PEG) chain and the polypropylene glycol (PPG) chain. The polyether-modified silicone-type surfactant may also have a C₈₋₂₂ alkyl chain introduced by modification. The usability of the cosmetic emulsion composition tends to be enhanced and the emulsion stability also tends to be increased by the incorporation of a polyether-modified silicone-type surfactant in the cosmetic emulsion composition. The cosmetic emulsion composition may contain a single polyether-modified silicone-type surfactant or may contain two or more. The polyether-modified silicone-type surfactant does not include species that correspond to crosslinked organopolysiloxane polymers.

The polyether-modified silicone-type surfactant is specifically exemplified by PEG-11 methyl ether dimethicone, PEG/PPG-20/22 butyl ether dimethicone, PEG/PPG-19/19 dimethicone, PEG-9 dimethicone, PEG-9 methyl ether dimethicone, PEG-10 dimethicone, PEG-21 methyl ether dimethicone, PEG-9 polydimethylsiloxyethyl dimethicone, and lauryl PEG-9 polydimethylsiloxyethyl dimethicone. From the standpoint of the usability and use sensation of the cosmetic emulsion composition, the polyether-modified silicone-type surfactant is more preferably at least one selected from PEG-11 methyl ether dimethicone, PEG/PPG-20/22 butyl ether dimethicone, and PEG/PPG-19/19 dimethicone, while PEG/PPG-19/19 dimethicone is still more preferred.

The value of the hydrophilic-lipophilic balance (HLB) for the polyether-modified silicone-type surfactant is, from the standpoint of the emulsion stability of the cosmetic emulsion composition, preferably 1 to 10 and more preferably 2 to 8. In the present specification, the HLB value of the polyether-modified silicone-type surfactant is the value determined by Griffin's method from the molecular weight and the number of ethylene oxide groups. When a commercial product is used, the HLB value provided in, for example, the corresponding catalogue can be directly used.

The content of the polyether-modified silicone-type surfactant that is component (C) is not particularly limited; however, from the standpoint of the usability and use sensation of the cosmetic emulsion composition, its content, with reference to the overall amount of the cosmetic emulsion composition, is preferably 0.01 to 4.0 mass%, more preferably 0.1 to 3.0 mass%, even more preferably 0.2 to 2.0 mass%, and still more preferably 0.5 to 1.5 mass%.

When the cosmetic emulsion composition contains a component (C), and viewed from the standpoint of bringing about a dramatic enhancement in the usability and use sensation of the cosmetic emulsion composition through the co-use of component (C) with component (A), the mass ratio between the component (A) content and the component (C) content in the cosmetic emulsion composition, expressed per 1 mass part of component (A), is component (C) at preferably 0.01 to 5 mass parts, more preferably 0.1 to 2 mass parts, still more preferably 0.2 to 1 mass parts, and even more preferably 0.3 to 0.8 mass parts.

In addition, viewed from the standpoint of bringing about a dramatic enhancement in the usability and use sensation of the cosmetic emulsion composition through the co-use of component (C) with component (B), the mass ratio between the component (B) content and the component (C) content in the cosmetic emulsion composition, expressed per 1 mass part of component (B), is component (C) at preferably 0.1 to 10 mass parts, more preferably 0.2 to 5 mass parts, still more preferably 0.4 to 3 mass parts, and even more preferably 0.5 to 2 mass parts.

The cosmetic emulsion composition according to the present invention may also contain glycerol. When the cosmetic emulsion composition does contain glycerol, the glycerol content is, with reference to the overall amount of the cosmetic emulsion composition, preferably 1 to 40 mass%, more preferably 5 to 35 mass%, and still more preferably 10 to 30 mass%. The incorporation of glycerol in the cosmetic emulsion composition is preferred because this improves the transparency of the cosmetic emulsion composition in particular, while the incorporation of more than 40 mass% is disfavored because this can cause a decline in the use sensation.

The cosmetic emulsion composition according to the present invention may also contain a lower alcohol. The lower alcohol can be exemplified by ethanol, propanol, isopropanol, butanol, and so forth. When the cosmetic emulsion composition does contain a lower alcohol, the lower alcohol content is preferably 0.1 to 20 mass%, more preferably 1 to 15 mass%, and still more preferably 2 to 10 mass%. The incorporation of ethanol in the indicated range as the lower alcohol is preferred for the present invention because this makes it possible to obtain a cosmetic emulsion composition having a particularly good use sensation.

The cosmetic emulsion composition according to the present invention preferably contains glycerol and ethanol from the standpoint of achieving an increase in particular in the usability and use sensation of the cosmetic emulsion composition. In this case, the mass ratio among component (A), glycerol, and ethanol in the cosmetic emulsion composition, expressed per 1 mass part of component (A), is preferably 1 to 40 mass parts glycerol and 0.5 to 10 mass parts ethanol, more preferably 2 to 20 mass parts glycerol and 1 to 5 mass parts ethanol, and still more preferably 5 to 15 mass parts glycerol and 2 to 4 mass parts ethanol.

The mass ratio in the cosmetic emulsion composition according to the present invention between the total mass of the components constituting the aqueous phase and the total mass of the components constituting the oil phase is not particularly limited, but this mass ratio is preferably 10 : 90 to 95 : 5 (the total for the mass ratio is 100). For the cosmetic emulsion composition according to the present invention, the total mass of the components constituting the oil phase can be calculated as the total mass of the various oily components, such as those described below; the total mass of the components (aqueous components) constituting the aqueous phase can be determined by subtracting the total mass of the oily components from the total mass of the cosmetic emulsion composition; and the resulting masses can then be used to determine the mass ratio between the total mass of the components constituting the aqueous phase and the total mass of the components constituting the oil phase. The presence of component (A) and component (B) in the cosmetic emulsion composition according to the present invention can bring about enhancements in particular in the usability, use sensation, and transparency of appearance by having the mass ratio between the total mass of the components constituting the aqueous phase and the total mass of the components constituting the oil phase be in the indicated range in the present invention.

The components constituting the oil phase of the cosmetic emulsion composition according to the present invention may be, without particular limitation, any oily components generally used in cosmetics, and this indicates solid, semisolid, and liquid (e.g., animal oils, plant oils, mineral oils, synthetic oils) oily components. These oily components can be exemplified by the fats and oils, waxes, hydrocarbon oils, ester oils, fluorinated oils, silicone oils, higher fatty acids, higher alcohols, and so forth that are generally used in ordinary cosmetics.

The fats and oils can be exemplified by the following: avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, Japanese nutmeg oil, rice bran oil, China wood oil, Japanese tung oil, jojoba oil, germ oil, triglycerin, cacao fat, coconut oil, hardened coconut oil, palm oil, palm kernel oil, Rhus succedanea seed oil, hardened oil, Japan wax, hardened castor oil, rosemary oil, camomile oil, eucalyptus oil, rice germ oil, γ-oryzanol, plant ceramides (glycosyl ceramides), carrot oil, Coix seed extract, Equisetum arvense extract, arnica extract, chamomile extract, Lithospermum erythrorhizone extract, linden extract, Achillea millefolium extract, sedge extract, Japanese Angelica root extract, horse chestnut extract, peach leaf extract, rosemary extract, Coix lacryma jobi extract, loquat extract, borage oil, and evening primrose oil.

The waxes can be exemplified by beeswax, candelilla wax, cotton wax, carnauba wax, bayberry wax, Chinese wax, spermaceti, montan wax, rice bran wax, lanolin, kapok wax, lanolin acetate, liquid lanolin, sugarcane wax, lanolin fatty acid isopropyl esters, hexyl laurate, reduced lanolin, jojoba wax, hard lanolin, shellac oil, POE lanolin alcohol ethers, POE lanolin alcohol acetate, lanolin fatty acid polyethylene glycol esters, and POE hydrogenated lanolin alcohol ethers.

The hydrocarbon oils can be exemplified by liquid paraffin, ozokerite, squalane, pristane, paraffin, ceresin, squalene, vaseline, and microcrystalline wax.

The ester oils can be exemplified by synthetic esters and esters between higher alcohols and higher fatty acids and can be specifically exemplified by diisobutyl adipate, isostearyl isostearate, cetyl 2-ethylhexanoate, isopropyl myristate, glyceryl trioctanoate, distearyl triisosterate, and glyceryl caprate.

The fluorinated oils can be exemplified by perfluorodecane, perfluorooctane, and perfluoropolyethers.

The silicone oil may be a liquid, gel, or swellable silicone compound, but is not otherwise particularly limited, and can be exemplified by chain polysiloxanes, cyclic polysiloxanes, amino-modified polysiloxanes, alkyl-modified polysiloxanes, polyether-modified silicones, and dimethicone crosspolymers. Examples at a more specific level are dimethylpolysiloxane (dimethicone), methylphenylpolysiloxane, methylhydrogenpolysiloxane, cyclopentasiloxane, hexamethylcyclotrisiloxane, octamethylcyclotetrasiloxane, decamethylcyclopentasiloxane, dodecamethylcyclohexasiloxane, tetramethyltetrahydrogencyclotetrasiloxane, phenyltrimethicone, diphenyldimethicone, phenyldimethicone, caprylylmethicone, lauryldimethicone/vinyldimethicone crosspolymer, dimethicone/vinyldimethicone crosspolymer, and dimethicone/phenylvinyldimethicone crosspolymer.

The higher fatty acids can be exemplified by lauric acid, myristic acid, palmitic acid, stearic acid, behenic acid, oleic acid, undecylenic acid, tall oil fatty acids, isostearic acid, linoleic acid, linolenic acid, eicosapentaenoic acid (EPA), and docosahexaenoic acid (DHA).

The higher alcohols can be exemplified by monohydric alcohols having a C₈₋₃₀ hydrocarbon group (straight chain or branched chain, saturated or unsaturated). Examples at a more specific level are caprylyl alcohol, capryl alcohol, lauryl alcohol, myristyl alcohol, cetyl alcohol, hexyldecanol, stearyl alcohol, isostearyl alcohol, oleyl alcohol, octyldodecanol, chimyl alcohol, decyltetradecanol, arachidyl alcohol, behenyl alcohol, carnaubyl alcohol, and ceryl alcohol.

One or two or more of the aforementioned oily components may be used for the oily component that constitutes the oil phase in the present invention. The oily component constituting the oil phase preferably includes a silicone oil based on a consideration of the usability and use sensation of the cosmetic emulsion composition according to the present invention. Among the aforementioned silicone oils, at least one selected from chain polysiloxanes and cyclic polysiloxanes is more preferably present based on a consideration of the usability and use sensation, while the incorporation of at least one chain polysiloxane and at least one cyclic polysiloxane is more preferred. When at least one chain polysiloxane and at least one cyclic polysiloxane are incorporated, the content ratio between the chain polysiloxane and cyclic polysiloxane is not particularly limited, but, with reference to 1 mass part of the chain polysiloxane, for example, the cyclic polysiloxane may be 0.1 to 10 mass parts, while the cyclic polysiloxane may be 0.2 to 5 mass parts based on a consideration of the usability and use sensation.

There are no particular limitations on the silicone oil content when the cosmetic emulsion composition contains a silicone oil; however, from the standpoint of the usability and use sensation of the cosmetic emulsion composition, the silicone oil content, with reference to the overall amount of the cosmetic emulsion composition, is preferably 1 to 30 mass%, more preferably 5 to 25 mass%, and still more preferably 10 to 25 mass%. Also in this case, and based on a consideration of the use sensation of the cosmetic emulsion composition, the mass percentage of the silicone oil in the total mass of the oil phase component in the cosmetic emulsion composition is preferably 20% to 100%, more preferably 40% to 100%, still more preferably 60% to 100%, and particularly preferably 80% to 100%.

The form of the cosmetic emulsion composition according to the present invention should be an emulsion, but is not otherwise particularly limited, and examples here are water-in-oil types, oil-in-water types, oil-water-oil types, and water-oil-water types. Based on a consideration of particularly enabling the expression of the effects of the present invention due to the incorporation of component (A) and component (B), a water-in-oil emulsion is preferred for the form of the cosmetic emulsion composition. When the cosmetic emulsion composition according to the present invention is a water-in-oil emulsion, and from the standpoint of achieving an increase in particular in the usability and use sensation of the cosmetic emulsion composition due to the incorporation of component (A) and component (B), the mass ratio between the total mass of the components constituting the aqueous phase and the total mass of the components constituting the oil phase is more preferably 40 : 60 to 95 : 5 (the total for the mass ratio is 100), even more preferably 60 : 40 to 90 : 10 (the total for the mass ratio is 100), and still more preferably 70 : 30 to 85 : 15 (the total for the mass ratio is 100).

The cosmetic emulsion composition according to the present invention may incorporate, in accordance with the particular objective, any other component that is commonly used in cosmetics. This any component that is commonly used in cosmetics can be exemplified by antimicrobial preservatives (excluding monoalkyl glyceryl ethers having a C₆₋₈ alkyl group), surfactants (excluding polyether-modified silicone-type surfactants), powder components, moisturizing agents, water-soluble polymer compounds, metal ion sequestrants, sugars, amino acids and derivatives thereof, organic amines, pH modifiers, vitamins, antioxidants, ultraviolet absorbers, fragrances, cosmetic components, blood circulation promoters, anti-inflammatory agents, stimulants and activators, antiseborrheic agents, anti-inflammatory agents, and other extracts, and any one or any two or more of these may be incorporated.

The antimicrobial preservatives can be exemplified by benzoic acid, salicylic acid, carbolic acid, sorbic acid, potassium sorbate, para-oxybenzoate esters, sodium benzoate, para-chloro-meta-cresol, hexachlorophene, benzalkonium chloride, chlorhexidine chloride, trichlorocarbanilide, photosensitizers, chlorphenesin, phenoxyethanol, methylparaben, ethylparaben, butylparaben, caprylyl glycol, resorcinol, triclosan, isopropylmethylphenol (IPMP), bis(2-pyridylthio-1-oxide)zinc, alkyldiaminoethylglycine hydrochloride, piroctone olamine, hinokitiol, vitamin B6 hydrochloride (pyridoxine hydrochloride), phenol, lysozyme hydrochloride, and cetylpyridinium chloride (CPC). One or two types selected from phenoxyethanol and caprylyl glycol are preferred among the preceding because this provides a low skin irritancy, a high safety for humans, and a substantial manifestation of the effects of the present invention.

The surfactant excludes polyether-modified silicone-type surfactants, but is otherwise exemplified by various anionic surfactants, cationic surfactants, nonionic surfactants, and amphoteric surfactants, and one or two or more of these may be used. The nonionic surfactants can be exemplified by sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate), glycerol fatty acid/polyglycerol fatty acids (for example, glycerol mono-cottonseed oil fatty acids, glycerol monoerucate, glycerol sesquioleate, glycerol monostearate, glycerol **α,α'**-oleate pyroglutamate, and glycerol monostearate malate), propylene glycol fatty acid esters (for example, propylene glycol monostearate), hardened castor oil derivatives, glycerol alkyl ethers, POE-sorbitan fatty acid esters (for example, POE-sorbitan monooleate, POE-sorbitan monostearate, and POE-sorbitan tetraoleate), POE-sorbitol fatty acid esters (for example, POE-sorbitol monolaurate, POE-sorbitol monooleate, POE-sorbitol pentaoleate, and POE-sorbitol monostearate), POE-glycerol fatty acid esters (for example, POE-glycerol monostearate, POE-glycerol monoisostearate, and POE-glycerol triisostearate), POE-fatty acid esters (for example, POE-distearate, POE-monostearate, POE-monodioleate, and ethylene glycol distearate), POE-alkyl ethers (for example, POE-lauryl ether, POE-oleyl ether, POE-stearyl ether, POE-behenyl ether, POE-2-octyldodecyl ether, and POE-cholestanol ether), Pluronic types (for example, Pluronic), POE·POP-alkyl ethers (for example, POE·POP-cetyl ether, POE·POP-2-decyltetradecyl ether, POE·POP-monobutyl ether, POE·POP-hydrogenated lanolin, and POE·POP-glycerol ether), tetra-POE·tetra-POP-ethylenediamine condensates (for example, Tetronic), POE-castor oil hardened castor oil derivatives (for example, POE-castor oil, POE-hardened castor oil, POE-hardened castor oil monoisostearate, POE-hardened castor oil triisostearate, POE-hardened castor oil monopyroglutamate monoisostearate diester, and POE-hardened castor oil maleate), POE-beeswax·lanolin derivatives (for example, POE-sorbitol beeswax), alkanolamides (for example, coconut oil fatty acid diethanolamides, lauric acid monoethanolamide, and fatty acid isopropanolamides), POE-propylene glycol fatty acid esters, POE-alkylamines, POE-fatty acid amides, sucrose fatty acid esters, alkylethoxydimethylamine oxide, and trioleyl phosphate.

The anionic surfactants can be exemplified by fatty acid soaps (for example, sodium laurate and sodium palmitate), higher alkyl sulfate ester salts (for example, sodium lauryl sulfate and potassium lauryl sulfate), alkyl ether sulfate ester salts (for example, POE-lauryl sulfate triethanolamine and sodium POE-lauryl sulfate), N-acylsarcosines (for example, sodium lauroylsarcosinate), higher fatty acid amide sulfonate salts (for example, sodium N-myristoyl-N-methyltaurate, sodium coconut oil fatty acid methyl taurate, and sodium lauryl methyl taurate), phosphate ester salts (sodium POE-oleyl ether phosphate, POE-stearyl ether phosphate, and so forth), sulfosuccinate salts (for example, sodium di-2-ethylhexyl sulfosuccinate, sodium monolauroyl monoethanolamide polyoxyethylene sulfosuccinate, and sodium lauryl polypropylene glycol sulfosuccinate), alkylbenzenesulfonate salts (for example, sodium linear dodecylbenzenesulfonate, triethanolamine salt of linear dodecylbenzenesulfonic acid, and linear dodecylbenzenesulfonic acid), higher fatty acid ester sulfate ester salts (for example, the sodium salt of hardened coconut oil fatty acid glycerol sulfate), N-acylglutamate salts (for example, monosodium N-lauroylglutamate, disodium N-stearoylglutamate, and monosodium N-myristoyl-L-glutamate), sulfated oils (for example, Turkey red oil), POE-alkyl ether carboxylic acids, POE-alkylallyl ether carboxylate salts, **α**-olefinsulfonate salts, higher fatty acid ester sulfonate salts, secondary alcohol sulfate ester salts, higher fatty acid alkylolamide sulfate ester salts, sodium lauroylmonoethanolamide succinate, ditriethanolamine salt of N-palmitoylaspartic acid, and sodium casein.

The cationic surfactant can be exemplified by alkyltrimethylammonium salts (for example, stearyltrimethylammonium chloride and lauryltrimethylammonium chloride), alkylpyridinium salts (for example, cetylpyridinium chloride), distearyldimethylammonium chloride, dialkyldimethylammonium salts, poly(N,N'-dimethyl-3,5-methylenepiperidinium) chloride, alkyl quaternary ammonium salts, alkyldimethylbenzylammonium salts, alkylisoquinolinium salts, dialkylmorpholinium salts, POE-alkylamines, alkylamine salts, polyamine fatty acid derivatives, amyl alcohol fatty acid derivatives, benzalkonium chloride, and benzethonium chloride.

The amphoteric surfactants can be exemplified by imidazoline-type amphoteric surfactants (for example, sodium 2-undecyl-N,N,N-(hydroxyethylcarboxymethyl)-2-imidazoline and 2-cocoyl-2-imidazolinium hydroxide-1-carboxyethyloxy disodium salt) and betaine-type surfactants (for example, 2-heptadecyl-N-carboxymethyl-N-hydroxyethylimidazolinium betaine, lauryldimethylaminoacetic acid betaine, alkyl betaines, amide betaines, and sulfo betaines).

The powder component can be exemplified by inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, silica, zeolites, barium sulfate, calcined calcium sulfate (exsiccated gypsum), calcium phosphate, fluorine apatite, hydroxyapatite, ceramic powders, metal soaps (for example, zinc myristate, calcium palmitate, and aluminum stearate), and boron nitride), organic powders (for example, polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder), inorganic white pigments (for example, titanium dioxide and zinc oxide), inorganic red pigments (for example, iron oxide (bengara) and iron titanate), inorganic brown pigments (for example, γ-iron oxide), inorganic yellow pigments (for example, iron oxide yellow and yellow ocher), inorganic black pigments (for example, iron oxide black and low-order titanium oxide), inorganic purple pigments (for example, manganese violet and cobalt violet), inorganic green pigments (for example, chromium oxide, chromium hydroxide, and cobalt titanate), inorganic blue pigments (for example, ultramarine and Prussian blue), pearlescent pigments (for example, titanium oxide-coated mica, titanium oxide-coated bismuth oxychloride, titanium oxide-coated talc, colored titanium oxide-coated mica, bismuth oxychloride, and fish scale foil), metal powder pigments (for example, aluminum powder and copper powder), organic pigments such as zirconium, barium, and aluminum lakes (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, as well as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1), and natural colorants (for example, chlorophyll and **β-**carotene).

The moisturizing agent can be exemplified by polyethylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salts, dl-pyrrolidonecarboxylate salts, short-chain soluble collagen, chestnut rose extract, yarrow extract, and melilot extract.

The water-soluble polymer compounds can be exemplified by starch polymers (for example, carboxymethylstarch and methylhydroxypropylstarch), cellulosic polymers (methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powder, and so forth), alginic acid polymers (for example, sodium alginate and the propylene glycol ester of alginic acid), vinyl polymers (for example, polyvinyl alcohol, polyvinyl methyl ether, polyvinylpyrrolidone, and carboxyvinyl polymers), polyoxyethylene polymers (for example, polyoxyethylene-polyoxypropylene copolymers obtained from polyethylene glycol 20,000, 40,000, or 60,000), acrylic polymers (for example, sodium polyacrylate, polyethyl acrylate, and polyacrylamide), polyethyleneimine, and cationic polymers.

The metal ion sequestrants can be exemplified by 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium meta-phosphate, gluconic acid, phosphoric acid, citric acid, ascorbic acid, succinic acid, edetic acid, and trisodium ethylenediaminehydroxyethyltriacetate.

The monosaccharides can be exemplified by trioses (for example, D-glyceryl aldehyde and dihydroxyacetone), tetroses (for example, D-erythrose, D-erythrulose, D-threose, and erythritol), pentoses (for example, L-arabinose, D-xylose, L-lyxose, D-arabinose, D-ribose, D-ribulose, D-xylulose, and L-xylulose), hexoses (for example, D-glucose, D-talose, D-psicose, D-galactose, D-fructose, L-galactose, L-mannose, and D-tagatose), heptoses (for example, aldoheptose and hepulose), octoses (for example, octulose), deoxysaccharides (for example, 2-deoxy-D-ribose, 6-deoxy-L-galactose, and 6-deoxy-L-mannose), aminosaccharides (for example, D-glucosamine, D-galactosamine, sialic acid, aminouronic acid, and muramic acid), and uronic acids (for example, D-glucuronic acid, D-mannuronic acid, L-guluronic acid, D-galacturonic acid, and L-iduronic acid).

The oligosaccharides can be exemplified by sucrose, umbelliferose, lactose, planteose, isolichnoses, **α,α**-trehalose, raffinose, lichnoses, umbilicin, stachyose, and verbascoses.

The polysaccharides can be exemplified by cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, keratosulfate, locust bean gum, succinoglucan, and charonic acid.

The amino acids can be exemplified by the neutral amino acids (for example, threonine and cysteine) and basic amino acids (for example, hydroxylysine). The amino acid derivatives can be exemplified by sodium acylsarcosinate (sodium lauroylsarcosinate), acylglutamate salts, sodium acyl-**β**-alanine, glutathione, and pyrrolidonecarboxylic acid.

The organic amines can be exemplified by monoethanolamine, diethanolamine, triethanolamine, morpholine, triisopropanolamine, 2-amino-2-methyl-1,3-propanediol, and 2-amino-2-methyl-1-propanol.

The pH modifiers can be exemplified by buffers, for example, lactic acid-sodium lactate, citric acid-sodium citrate, succinic acid-sodium succinate.

The vitamins can be exemplified by vitamin E and derivatives thereof, vitamin C and derivatives thereof, vitamin D and derivatives thereof, vitamin F and derivatives thereof, vitamin K and derivatives thereof, vitamin A and derivatives thereof, and vitamin B derivatives; however, there is no limitation to these. Specific examples are **γ-**tocopherol; stearyl ascorbate; ascorbyl dipalmitate; tocopherol nicotinate; menadione; dehydrocholesterol; ergocalciferol; pyridoxine dicaprylate; ascorbyl tetrahexyldecanoate (VCIP); retinol derivatives such as retinol, retinol palmitate, and retinol acetate; docosahexaenoic acid; linoleic acid; pantenol; tocopherol linoleate; isopropyl linoleate; linolenic acid; pyridoxine palmitate; vitamin A; **β**-carotene; pyridoxine dipalmitate; phylloquinone; pantothenic acid and derivatives thereof; and biotin.

The antioxidants can be exemplified by dibutylhydroxytoluene, butylhydroxyanisole, sorbic acid, sodium sulfite, sodium bisulfite, sodium thiosulfate, metabisulfite salts, thiotaurine, hypotaurine, thioglycerol, thiourea, thioglycolic acid, cysteine hydrochloride, propyl gallate, gallic acid derivatives, ascorbic acid, ascorbic acid derivatives (ascorbic acid phosphoric acid esters and so forth), tocopherol, tocopherol derivatives, erythorbic acid, p-t-butylphenol, phytic acid, and L-cysteine hydrochloride.

The ultraviolet absorbers can be exemplified by benzoic acid-type ultraviolet absorbers, anthranilic acid-type ultraviolet absorbers, salicylic acid-type ultraviolet absorbers, cinnamic acid-type ultraviolet absorbers, benzophenone-type ultraviolet absorbers, benzotriazole-type ultraviolet absorbers, triazine-type ultraviolet absorbers, benzoate-type ultraviolet absorbers, cyanoacrylate-type ultraviolet absorbers, oxanilide-type ultraviolet absorbers, and formamidine-type ultraviolet absorbers.

The benzoic acid-type ultraviolet absorbers can be exemplified by para-aminobenzoic acid, ethyl para-aminobenzoate, ethylhexyl para-dimethylaminobenzoate, octyl para-dimethylaminobenzoate, amyl para-dimethylaminobenzoate, para-aminobenzoic acid monoglyceride, glyceryl para-aminobenzoate, ethyldihydroxypropyl para-aminobenzoate, ethyl N,N-dipropoxy-para-aminobenzoate, ethyl N,N-diethoxy-para-aminobenzoate, ethyl N,N-dimethyl-para-aminobenzoate, butyl N,N-dimethyl-para-aminobenzoate, amyl N,N-dimethyl-para-aminobenzoate, octyl N,N-dimethyl-para-aminobenzoate, and hexyl diethylaminohydroxybenzoylbenzoate. The anthranilic acid-type ultraviolet absorbers can be exemplified by homomenthyl N-acetylanthranilate.

The salicylic acid-type ultraviolet absorbers can be exemplified by salicylic acid and the sodium salt thereof, amyl salicylate, menthyl salicylate, homomenthyl salicylate, octyl salicylate, phenyl salicylate, benzyl salicylate, and p-isopropanolphenyl salicylate. The cinnamic acid-type ultraviolet absorbers can be exemplified by octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, 2-ethylhexyl p-methoxycinnamate (2-ethylhexyl ester of para-methoxycinnamic acid), 2-ethoxyethyl p-methoxycinnamate (cinoxate), cyclohexyl p-methoxycinnamate, ethyl **α**-cyano-**β**-phenylcinnamate, 2-ethylhexyl **α**-cyano-**β**-phenylcinnamate (octocrylene), glyceryl mono-2-ethylhexanoyl di-para-methoxy cinnamate, and ferulic acid and derivatives thereof.

The benzophenone-type ultraviolet absorbers can be exemplified by 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzoin-3), 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, 4-hydroxy-3-carboxybenzophenone, and 5,5'-methylenebis(2-hydroxy-4-methoxybenzophenone).

The benzotriazole-type ultraviolet absorbers can be exemplified by 2-(2-hydroxy-5-methylphenyl)benzotriazole, 2-(2-hydroxy-5-methylphenyl)-5-chlorobenzotriazole, 2-(2-hydroxy-5-tertiary-octylphenyl)benzotriazole, 2-(2-hydroxy-5-tertiary-octylphenyl)-5-chlorobenzotriazole, 2-(2-hydroxy-3,5-di-tertiary-butylphenyl)benzotriazole, 2-(2-hydroxy-3,5-di-tertiary-butylphenyl)-5-chlorobenzotriazole, 2-(2-hydroxy-3-tertiary-butyl-5-methylphenyl)benzotriazole, 2-(2-hydroxy-3-tertiary-butyl-5-methylphenyl)-5-chlorobenzotriazole, 2-(2-hydroxy-3,5-dicumylphenyl)benzotriazole, 2-(2-hydroxy-3,5-dicymylphenyl)-5-chlorobenzotriazole, 2,2'-methylenebis(4-tertiary-octyl-6-benzotriazolylphenol), the polyethylene glycol ester of 2-(2-hydroxy-3-tertiary-butyl-5-carboxyphenyl)benzotriazole, 2-[2-hydroxy-3-(2-acryloyloxyethyl)-5-methylphenyl]benzotriazole, 2-[2-hydroxy-3-(2-acryloyloxyethyl)-5-methylphenyl]-5-chlorobenzotriazole, 2-[2-hydroxy-3-(2-methacryloyloxyethyl)-5-tertiary-butylphenyl]benzotriazole, 2-[2-hydroxy-3-(2-methacyloyloxyethyl)-5-tertiary-butylphenyl]-5-chlorobenzotriazole, 2-[2-hydroxy-3-(2-methacryloyloxyethyl)-5-tertiary-octylphenyl]benzotriazole, 2-[2-hydroxy-3-(2-methacryloyloxyethyl)-5-tertiary-octylphenyl]-5-chlorobenzotriazole, 2-[2-hydroxy-3-(2-methacryloyloxyethyl)-5-tertiary-butylphenyl]benzotriazole, 2-[2-hydroxy-3-(2-methacryloyloxyethyl)-5-tertiary-butylphenyl]-5-chlorobenzotriazole, 2-[2-hydroxy-5-(2-methacryloyloxyethyl)phenyl]benzotriazole, 2-[2-hydroxy-5-(2-methacryloyloxyethyl)phenyl]-5-chlorobenzotriazole, 2-[2-hydroxy-3-tertiary-butyl-5-(2-methacryloxyoxyethyl)phenyl]benzotriazole, 2-[2-hydroxy-3-tertiary-butyl-5-(2-methacryloxyoxyethyl)phenyl]-5-chlorobenzotriazole, 2-[2-hydroxy-3-tertiary-amyl-5-(2-methacryloyloxyethyl)phenyl]benzotriazole, 2-[2-hydroxy-3-tertiary-amyl-5-(2-methacryloyloxyethyl)phenyl]-5-chlorobenzotriazole, 2-[2-hydroxy-3-tertiary-butyl-5-(3-methacryloxyloxypropyl)phenyl]benzotriazole, 2-[2-hydroxy-3-tertiary-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(2-methacryloyloxymethyl)phenyl]-5-chlorobenzotriazole, 2-[2-hydroxy-4-(3-methacryloyloxy-2-hydroxypropyl)phenyl]benzotriazole, 2-[2-hydroxy-4-(3-methacryloyloxy-2-hydroxypropyl)phenyl]-5-chlorobenzotriazole, 2-[2-hydroxy-4-(3-methacryloyloxypropyl)phenyl]benzotriazole, and 2-[2-hydroxy-4-(3-methacryloyloxypropyl)phenyl]-5-chlorobenzotriazole.

The triazine-type ultraviolet absorbers can be exemplified by 2,4,6-tris[4-(2-ethylhexyloxycarbonyl)anilino]-1,3,5-triazine, 2,4-bis[{4-(2-ethylhexyloxy)-2-hydroxy}phenyl]-6-(4-methoxyphenyl)-1,3,5-triazine, 2-(2-hydroxy-4-methoxyphenyl)-4,6-diphenyl-1,3,5-triazine, 2-(2-hydroxy-4-hexyloxyphenyl)-4,6-diphenyl-1,3,5-triazine, 2-(2-hydroxy-4-octoxyphenyl)-4,6-bis(2,4-dimethylphenyl)-1,3,5-triazine, 2-[2-hydroxy-4-(3-mixed C12-C13-alkoxy-2-hydroxypropoxy)phenyl]-4,6-bis(2,4-dimethylphenyl)-1,3,5-triazine, 2-[2-hydroxy-4-(2-acryloyloxyethoxy)phenyl]-4,6-bis(4-methylphenyl)-1,3,5-triazine, 2-[2-hydroxy-4-(2-acetyloxyethoxy)phenyl]-4,6-bisphenyl-1,3,5-triazine, 2-(2,4-dihydroxy-3-allylphenyl)-4,6-bis(2,4-dimethylphenyl)-1,3,5-triazine, and 2,4,6-tris(2-hydroxy-3-methyl-4-hexyloxyphenyl)-1,3,5-triazine. The benzoate ultraviolet absorbers can be exemplified by resorcinol monobenzoate, 2,4-di-tertiary-butylphenyl 3,5-di-tertiary-butyl-4-hydroxybenzoate, octyl (3,5-di-tertiary-butyl-4-hydroxy)benzoate, dodecyl (3,5-di-tertiary-butyl-4-hydroxy)benzoate, tetradecyl (3,5-di-tertiary-butyl-4-hydroxy)benzoate, hexadecyl (3,5-di-tertiary-butyl-4-hydroxy)benzoate, octadecyl (3,5-di-tertiary-butyl-4-hydroxy)benzoate, behenyl (3,5-di-tertiary-buty-4-hydroxy)benzoate, and stearyl (3,5-di-tertiary-butyl-4-hydroxy)benzoate.

The cyanoacrylate-type ultraviolet absorbers can be exemplified by ethyl **α**-cyano-**β,β**-diphenylacrylate and methyl 2-cyano-3-methyl-3-(p-methoxyphenyl)acrylate. The oxanilide-type ultraviolet absorbers can be exemplified by 2-ethyl-2'-ethoxyoxanilide and 2-ethoxy-4'-dodecyloxanilide. The formamidine-type ultraviolet absorbers can be exemplified by N,N'-diphenyl-N'-(4-ethoxycarbonylphenyl)formamidine, N'-(4-ethoxycarbonylphenyl)-N-methyl-N-phenylformamidine, N,N'-bis(4-ethoxycarbonylphenyl)-N-methylformamidine, N'-(4-ethoxycarbonylphenyl)-N-(2'-methoxyphenyl)-N-methylformamidine, and N-(4-n-butoxycarbonylphenyl)-N'-(4'-ethylcarbonyl)-N-methylformamidine.

Other ultraviolet absorbers can be exemplified by 3-(4'-methylbenzylidene)-d,l-camphor, 3-benzylidene-d,l-camphor, 2-phenyl-5-methylbenxoxazole, dibenzalazine, dianisoylmethane, 5-(3,3-dimethyl-2-norbornylidene)-3-pentane-2-one, 4-t-butylmethoxydibenzoylmethane, octyltriazone, urocanic acid, ethyl urocanate, 1-(3,4-dimethoxyphenyl)-4,4-dimethyl-1,3-pentanedione, 2-ethylhexyl dimethoxybenzylidenedioxoimidazolidinepropionate, phenylbenzimidazolesulfonic acid, terephthalylidene dicamphor sulfonic acid, drometrizole trisiloxane, methyl anthranilate, rutin, rutin derivatives, oryzanol, and oryzanol derivatives.

The fragrances can be exemplified by compound fragrances containing natural fragrances and/or synthetic fragrances. Specific examples of natural fragrances are amyris oil, ambrette seed oil, ylang ylang oil, ylang ylang absolute, iris resinoid, iris absolute, iris oil, wintergreen oil, estragon oil, elemi oreoresin, elemi resinoid absolute, elemi tincture, oakmoss concrete, oakmoss absolute, oakmoss resin, oakmoss resinoid, osmanthus absolute, osmanthus concrete, opopanax resinoid, opopanax absolute, opopanax oil, olibanum resinoid, olibanum absolute, olibanum oil, allspice oil, origanum oil, oregano oil, oregano oleoresin, orange flower absolute, orange flower concrete, cananga oil, gurjun balsam, gurjun balsam oil, cassie absolute, cassie flower oil, cassia oil, gardenia absolute, carnation absolute, cabreuva oil, chamomile oil, cardamom oil, galbanum oil, galbanum resin, galbanum resinoid, caraway seed oil, carrot seed oil, cubeba oil, guaiac wood oil, guaiac resin, guaiac concrete, camphor oil, cumin oil, cumin absolute, cumin oleoresin, clary sage oil, grapefruit oil, clove oil, costus oil, copaiba balsam, copaiba balsam oil, copaiba balsam resin, coriander oil, sandalwood oil, shiso oil, cedar wood oil, citronella oil, jasmine oil, jasmine absolute, jasmine concrete, juniper berry oil, perilla seed absolute, jonquil absolute, ginger oil, cinnamon oil, cinnamon bark oil, cinnamon leaf oil, Japanese cedarwood oil, star anise oil, styrax oil, styrax resinoid, spike lavender oil, spearmint oil, savory oil, sage oil, cedar oil, cedar leaf oil, geranium oil, celery seed oil, thyme oil, tagette oil, tangerine oil, tuberose absolute, tea tree oil, tree moss absolute, tonka bean oil, tolu balm, nutmeg oil, narcissus absolute, neroli oil, violet leaf absolute, pine oil, pine needle oil, basil oil, parsley oil, parsley seed oil, parsley herb oil, patchouli oil, mentha oil, vanilla absolute, honeysuckle absolute, palmarosa oil, valerian oil, bitter orange oil, hyssop oil, hiba oil, hinoki oil, hyacinth absolute, fennel oil, fig absolute, petitgrain oil, buchu oil, bay oil, vetiver oil, pepper oil, peppermint absolute, peppermint oil, bergamot oil, Peruvian balsam, benzoin tincture, benzoin resinoid, hosho oil, marjoram oil, mandarin oil, mandarin orange oil, mimosa concrete, mimosa absolute, mimosa oil, myrrh resinoid, myrrh absolute, myrrh oil, musk absolute, musk tincture, eucalyptus oil, yuzu oil, lime oil, labdanum oil, labdanam resinoid, lavender oil, lavender absolute, lavandin oil, lavandin absolute, lemon oil, lemongrass oil, rose oil, rose absolute, rose concrete, rosemary oil, laurel oil, and laurel leaf oil.

The synthetic fragrances can be exemplified by ambrettolide, C₆₋₁₂ aldehydes, anisaldehyde, acetal R, acetophenone, acetyl cedrene, adoxal, allyl amyl glycolate, allyl cyclohexanepropionate, ambroxane, amylcinnamic aldehyde, amylcinnamic aldehyde dimethyl acetal, amyl valerate, amyl salicylate, acetyleugenol, isoamyl acetate, isoamyl salicylate, indole, ionone, isobornyl acetate, isocyclocitral, Iso E Super, isoeugenol, isononyl acetate, isobutyl quinoline, **γ-**undecalactone, ethylene brassylate, ethylene dodecanedioate, ethylvanillin, 2-ethylhexanol, oranthiol, 10-oxahexadecanolide, 11-oxahexadecanolide, 12-oxahexadecanolide, oxahexadecen-2-one, eugenol, orivone, oxyphenylon, galaxolide, caryophyllene, cashmeran, carvone, **β**-caryophyllene, carone, coumarin, p-cresyl methyl ether, geraniol, geranyl acetate, geranyl formate, geranyl nitrile, koavone, sandalore, sandela, santalex, cinnamic alcohol, cinnamic aldehyde, cis jasmon, citral, citral dimethyl acetal, citrathal, citronellal, citronellol, citronellyl acetate, citronellyl formate, citronellyl nitrile, cyclacet, cyclamen aldehyde, cyclaprop, dimethylbenzyl carbinol, dihydrojasmone, dihydrolinalool, dihydromyrcenol, dimetol, dimyrcetol, diphenyl oxide, jasmal, jasmolactone, jasmophyllan, cinnamyl acetate, cyclopentadecanone, cyclohexadecenone, cyclopentadecanolide, cyclohexadecanolide, dimethylbenzylcarbinyl acetate, jasmacyclene, styralyl acetate, styralyl propionate, cedramber, cedryl acetate, cedrol, celestolide, **α**-damascone, **β**-damascone, **δ**-damascone, damascenone, terpineol, terpinyl acetate, thymol, tetrahydrolinalool, tetrahydrolinalyl acetate, tetrahydrogeraniol, tetrahydrogeranyl acetate, tonalid, traseolide, triplal, neryl acetate, nerol, neobergamate, **γ-**nonalactone, nopyl alcohol, nopyl acetate, bacdanol, hydrotropic alcohol, α-pinene, β-pinene, hydroxycitronellal, hyacinth dimethyl acetal, butyl butyrate, p-t-butyl cyclohexanol, p-t-butylcyclohexyl acetate, o-t-butylcyclohexanol, o-t-butylcyclohexyl acetate, fruitate, fenchyl alcohol, phenylethylphenyl acetate, phenylethyl acetate, pentalide, verdox, benzyl acetate, benzyl alcohol, benzyl salicylate, bergamyl acetate, benzaldehyde, benzyl formate, hedione, helional, heliotropin, cis-3-hexenol, cis-3-hexenyl acetate, cis-3-hexenyl salicylate, hexylcinnamic aldehyde, hexyl salicylate, bornyl acetate, borneol, manzanate, mayol, myrcene, myrac aldehyde, muguet aldehyde, mugol, musk TM-11, musk 781, musk C14, muscone, musk ketone, musk tibetine, menthanyl acetate, menthonate, methyl anthranilate, methyleugenol, menthol, **α**-methylionone, β-methylionone, **γ-**methylionone, methylisoeugenol, methyl lavender ketone, methyl salicylate, 14-methylhexadecenolide, 14-methylhexadecanolide, methyl naphthyl ketone, methylphenyl acetate, yara yara, **δ**-C₆₋₁₃ lactones, lime oxide, **γ**-C₆₋₁₃ lactones, raspberry ketone, limonene, ligustral, lilial, linalool, linalool oxide, linalyl acetate, lyral, rhubafuran, rosephenone, rose oxide, and vanillin.

The cosmetic ingredients can be exemplified by placenta extract, mulberry root bark extract, Saxifraga sarmentosa extract, perilla extract, white mustard seed extract and hydrolyzates thereof, fermented white mustard seed, rosa damascena extract, peony root extract and hydrolysates thereof, lactobacillus-fermented rice, lotus seed extract and hydrolyzates thereof, fermented lotus seed, ginseng extract, coix seed hydrolyzate, fermented coix seed, fermented royal jelly, fermented sake lees, Pandanus amaryllifolius extract, Arcangelicia flava Merrilli extract, kiwi fruit extract, chamomile extract, coral grass extract, rice leaf extract and hydrolyzates thereof, eggplant (e.g., mizunasu, long eggplant, Kamo eggplant, American eggplant, etc.) extract and hydrolyzates thereof, extracts of seaweeds such as Betaphycus gelatinum and so forth, extracts of marine flowering plants such as eelgrass and so forth, fermented soy milk, deep ocean water, fermented rice extract, linoleic acid and derivatives thereof and processed products thereof (e.g., liposomalized linoleic acid), collagen of animal or fish origin and derivatives thereof, elastin and derivatives thereof, glycyrrhizinic acid and derivatives thereof (e.g., dipotassium salt), t-cycloamino acid derivatives, allantoin, arbutin, diisopropylamine dichloroacetate, **γ**-amino-**β**-hydroxybutyric acid, gentian extract, licorice extract, carrot extract, aloe extract, Laminaria angustata extract, Ulva pertusa extract, juazeiro extract, and immature peach extract. A single one of these or two or more may be used.

The other blendable components can be exemplified by blood circulation promoters (for example, benzyl nicotinate, β-butoxyethyl nicotinate, capsaicin, zingerone, cantharide tincture, ichthammol, tannic acid, α-borneol, tocopherol nicotinate, inositol hexanicotinate, cyclandelate, cinnarizine, tolazoline, acetylcholine, verapamil, cepharanthine, and **γ-**oryzanol), inflammation-reducing agents (for example, glycyrrhizinic acid derivatives, glycyrrhetinic acid derivatives, salicylic acid derivatives, hinokitiol, zinc oxide, and allantoin), stimulants and activators (for example, royal jelly, photosensitizers, and cholesterol derivatives), antiseborrheic agents (for example, sulfur and thianthol), anti-inflammatory agents (for example, tranexamic acid, thiotaurine, and hypotaurine), and other extracts (for example, phellodendron bark, Coptis rhizome, Lithospermum erythrorhizon root, Paeonia lactiflora, Swertia herb, birch, sage, Japanese loquat, carrot, aloe, common mallow, iris, grape, coix seed, Luffa cylindrica, lily, saffron, Cnidium rhizome, pine strobile, hypericum erectum, ononis, garlic, red pepper, Citrus unshiu peel, Japanese Angelica root, and seaweed). A single one of these or two or more may be used.

There are no particular limitations on the method for producing the cosmetic emulsion composition according to the present invention, and known methods for producing emulsion compositions may be used. An example is a method in which an aqueous phase and oil phase are respectively prepared; the aqueous phase and oil phase are mixed, optionally while heated, followed by emulsification using an emulsifying device; and cooling to room temperature is then performed. In this method, known techniques may also be used to prepare the individual aqueous phase and oil phase, and the method for blending the individual components, i.e., component (A), component (B), and so forth, is also not limited.

There are no particular limitations on the formulation of the cosmetic emulsion composition according to the present invention, and it may be, for example, a liquid, gel, sherbet, milky lotion, cream, ointment, solid paste, paste, solid, or powder. The cosmetic creams can be exemplified by oil-free creams, which do not use an oil fraction; water-free creams, which do not use a water fraction; oily creams, which use an oil fraction in large amounts; weakly oily creams, which use a small amount of oil fraction; and medium oily creams, for which the oil fraction is between oily creams and weakly oily creams.

The viscosity of the cosmetic emulsion composition according to the present invention is not particularly limited and may be adjusted as appropriate in conformity with the particular application. For example, the viscosity at 25°C may be approximately 100 to 500,000 mPa·s. The viscosity at 25°C in the present invention is measured using a B-type viscometer. For example, when the cosmetic emulsion composition according to the present invention is a gel, and viewed from the standpoint of the usability and use sensation, the viscosity at 25°C is preferably 10,000 to 400,000 mPa·s, more preferably 50,000 to 300,000 mPa·s, and still more preferably 80,000 to 250,000 mPa·s.

There are no particular limitations on the cosmetics that can use the cosmetic emulsion composition according to the present invention, and examples here are the known cosmetics in which an emulsion composition is used. Specific examples of the cosmetics are skin care cosmetics such as lotions, cosmetic liquids, and emulsions; make-up cosmetics such as liquid foundations, make-up foundations, eye shadows, eyeliners, and lipsticks; and sunscreen cosmetics such as sunscreen liquids and sunscreen creams.

### Examples

The present invention is specifically described below using examples, but the present invention is in no way limited to or by these examples, and modifications and alterations may be made within a range in which there is no departure from the scope of the present invention. In the following examples and so forth, % is on a mass basis unless specifically indicated otherwise.

Hydrophobically Modified Polyether Urethane Production Example 1

402.2 g of polyethylene glycol (PEG-240) was introduced into a 1000-mL four-neck flask fitted with a thermometer, nitrogen introduction line, and stirrer and melting was carried out by heating to 50°C to 60°C. This was followed by the introduction of 11.7 g of hexamethylene diisocyanate and substitution of the interior with nitrogen. Stirring was performed until the components had become uniform, and, once it had been confirmed that the components had been mixed to uniformity, the temperature was raised to 70°C to 80°C and a reaction was run for 3 hours at the same temperature. This was followed by the addition to the system of 86.1 g of polyethylene glycol (20) mono-2-decyldodecyl ether, and the reaction was run for another 6 hours at 70°C to 80°C to obtain a hydrophobically modified polyether urethane.

The compounds of the components (A) to (C) used in the examples are as follows.

### Component (A)

A-1: cyclohexyl glyceryl ether

### Component (B)

B-1: the hydrophobically modified polyether urethane composition obtained in Production Example 1, which, with reference to general formula (I), is a mixture of d = 0 to 10 wherein R¹ and R⁸ are each the decyl group, R² and R⁹ are each the dodecyl group, R³, R⁵, and R⁷ are each the ethylene group, R⁴ and R⁶ are each the hexyl group, a = 20, b = 240, and c = 20 Component (C)

C-1: PEG/PPG-19/19 dimethicone (HLB: 2.0)

### Examples 1 and 2 and Comparative Examples 1 to 7

Using the blending conditions given in Table 1 (the numerical values indicate the amount blended (mass%) for each component), water-in-oil gel emulsions were produced for the cosmetic emulsion compositions. More specifically, the surfactant and oil phase components were heated and dissolved; the aqueous phase components were separately heated and dissolved; and the aqueous phase components were added to the oil phase components and emulsification was performed to obtain the cosmetic. The viscosity at 25°C of the resulting cosmetics was measured with a B-type viscometer, and these results are given in Table 1.

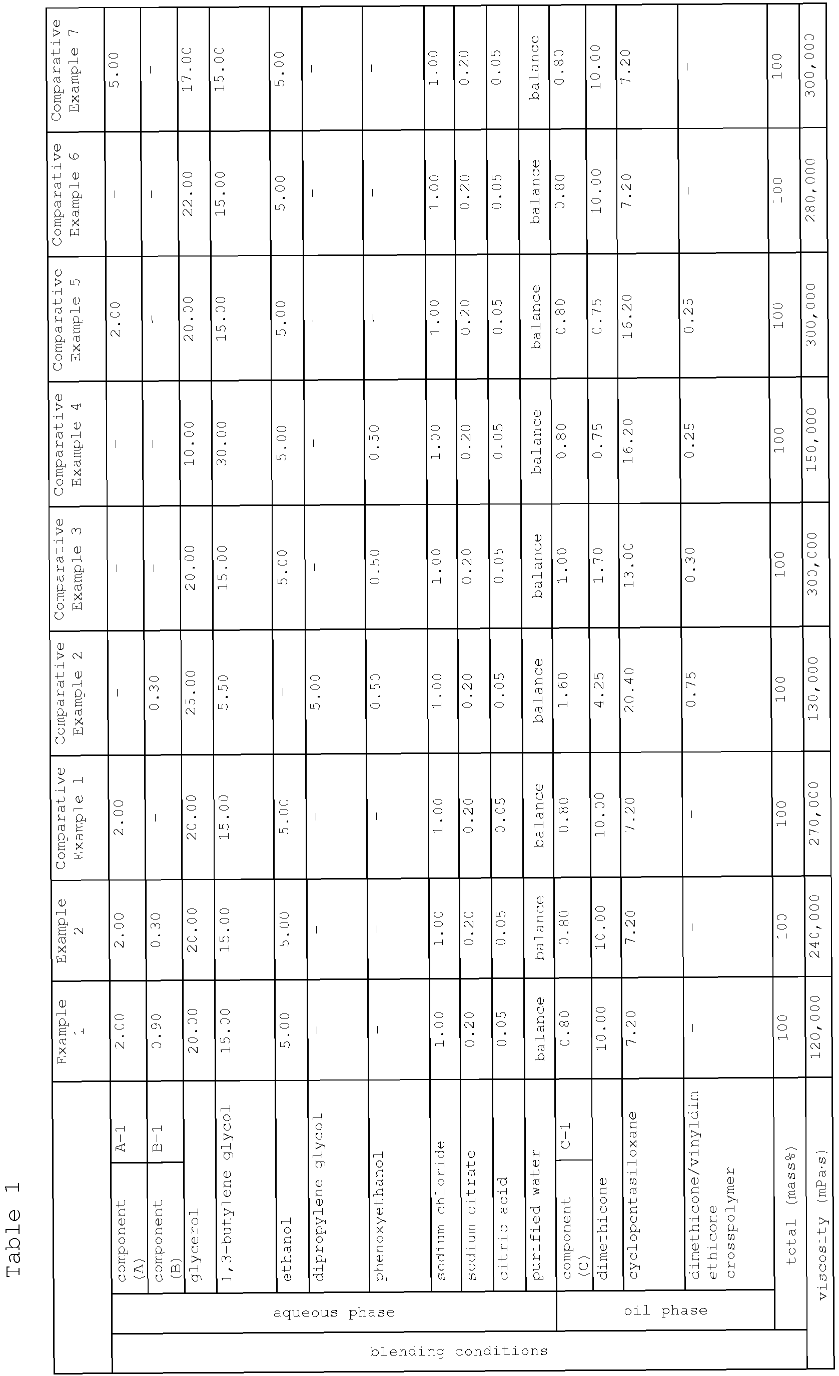

The obtained cosmetic emulsion compositions were submitted to an evaluation of the usability and use sensation by sensory testing. The evaluation was specifically conducted as follows: using a five-member panel, the cosmetic emulsion compositions were actually used on the skin, at which time the usability and use sensation were each evaluated based on the evaluation criteria provided below; the average score for the five panelists was used for the score for the usability and use sensation. The transparency of appearance of the obtained cosmetic emulsion compositions was also evaluated as well based on the criteria given below. The results are given in Table 2.

### Evaluation Criteria for Usability

Easily taken up with the fingers and spreads smoothly on the skin ... 5 points Somewhat difficult to take up with the fingers and difficult to smoothly spread on the skin ... 3 points Difficult to take up with the fingers and does not spread smoothly on the skin ... 1 point

Evaluation Criteria for Use Sensation The skin feels soft and a persistent moist sensation is perceived ... 5 points
The skin feels soft to some degree and a persistent moist sensation is perceived to some degree ... 3 points The skin feels tight and a persistent moist sensation is not perceived ... 1 point

Evaluation Criteria for Transparency of Appearance Very highly transparent ... O

### Cloudiness or turbidity is seen ... X

| | Example 1 | Example 2 | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| usability | 5.0 | 3.8 | 1.4 | 3.0 | 1.0 | 2.6 | 1.0 | 1.4 | 1.0 |
| use sensation | 4.6 | 4.2 | 2.6 | 2.6 | 1.4 | 1.8 | 2.2 | 2.2 | 3.8 |
| transparency of appearance | O | O | O | X | X | X | O | X | O |

The results demonstrate that the cosmetic emulsion composition according to the present invention has extremely good usability, use sensation, and transparency of appearance. Moreover, it is also expected that the cosmetic emulsion composition according to the present invention may have excellent antimicrobial performance because component (A) also functions as an antimicrobial preservative.

### INDUSTRIAL APPLICABILITY

The present invention relates to a cosmetic emulsion composition that exhibits excellent transparency and a usability and use sensation that are dramatically enhanced over that of conventional emulsion compositions, and can be used in a variety of cosmetic types and cosmetic formulations.

## Claims

1. A cosmetic emulsion composition comprising (A) a component containing a monoalkyl glyceryl ether having a C₆₋₈ alkyl group and (B) a component containing an associative urethane.

2. The cosmetic emulsion composition according to claim 1, wherein component (A) contains a cyclohexyl glyceryl ether.

3. The cosmetic emulsion composition according to claim 1 or 2, wherein the content of component (A) is 0.01 to 10 mass% with reference to the overall amount of the cosmetic emulsion composition.

4. The cosmetic emulsion composition according to any one of claims 1 to 3, wherein component (B) contains a hydrophobically modified polyether urethane having the following general formula (I): 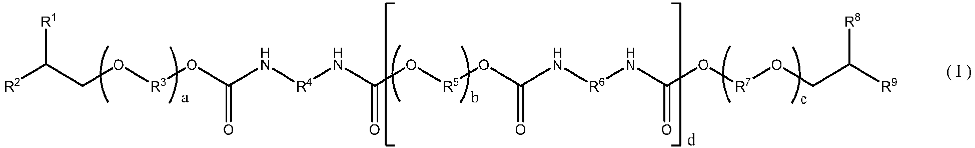 wherein, R¹, R², R⁸, and R⁹ each independently represent a C₁₋₂₀ hydrocarbon group; R³, R⁵, and R⁷ each independently represent a C₂₋₄ divalent hydrocarbon group; R⁴ and R⁶ each independently represent a C₃₋₁₆ divalent hydrocarbon group; a and c each independently represent a number from 10 to 100; b represents a number from 100 to 500; and d represents a number from 0 to 10.

5. The cosmetic emulsion composition according to any one of claims 1 to 4, wherein the content of component (B) is 0.01 to 5.0 mass% with reference to the overall amount of the cosmetic emulsion composition.

6. The cosmetic emulsion composition according to any one of claims 1 to 5, wherein component (B) is 0.01 to 5 mass parts per 1 mass part of component (A) in the cosmetic emulsion composition.

7. The cosmetic emulsion composition according to any one of claims 1 to 6, wherein the cosmetic emulsion composition additionally contains a polyether-modified silicone-type surfactant as a component (C).

8. The cosmetic emulsion composition according to any one of claims 1 to 7, wherein the mass ratio between the total mass of the components constituting the aqueous phase and the total mass of the components constituting the oil phase is 10 : 90 to 95 : 5 (the total for the mass ratio is 100).

9. The cosmetic emulsion composition according to any one of claims 1 to 8, wherein the oil phase of the cosmetic emulsion composition contains a silicone oil.

10. The cosmetic emulsion composition according to any one of claims 1 to 9, wherein the cosmetic emulsion composition is a water-in-oil emulsion.

11. The cosmetic emulsion composition according to any one of claims 4 to 10, wherein R¹ and R⁸ are each a decyl group; R² and R⁹ are each a dodecyl group; R³, R⁵, and R⁷ are each an ethylene group; R⁴ and R⁶ are each a hexyl group; a and c are each 20; and b is 240.

12. The use of an emulsion composition comprising (A) a component containing a monoalkyl glyceryl ether having a C₆₋₈ alkyl group and (B) a component containing an associative urethane for the manufacture of a cosmetic.

13. The use according to claim 12, wherein component (A) contains a cyclohexyl glyceryl ether.

14. The use according to claim 12 or 13, wherein component (B) contains a hydrophobically modified polyether urethane having the following general formula (I): 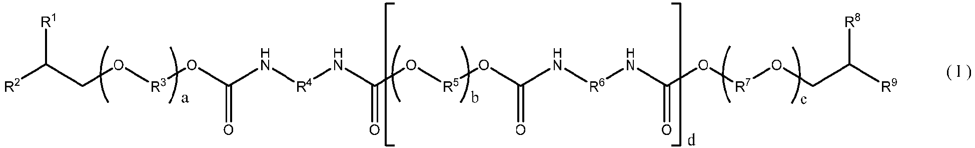 wherein, R¹, R², R⁸, and R⁹ each independently represent a C₁₋₂₀ hydrocarbon group; R³, R⁵, and R⁷ each independently represent a C₂₋₄ divalent hydrocarbon group; R⁴ and R⁶ each independently represent a C₃₋₁₆ divalent hydrocarbon group; a and c each independently represent a number from 10 to 100; b represents a number from 100 to 500; and d represents a number from 0 to 10.

15. The use according to any one of claims 12 to 14, wherein the emulsion composition further comprises a polyether-modified silicone-type surfactant as a component (C).

## Patentansprüche

1. Kosmetische Emulsionszusammensetzung, die (A) eine Komponente, die einen Monoalkylglycerylether enthält, der eine C₆₋₈-Alkylgruppe aufweist, und (B) eine Komponente umfasst, die ein assoziatives Urethan enthält.

2. Kosmetische Emulsionszusammensetzung nach Anspruch 1, wobei Komponente (A) einen Cyclohexylglycerylether enthält.

3. Kosmetische Emulsionszusammensetzung nach Anspruch 1 oder 2, wobei der Gehalt an Komponente (A) 0,01 bis 10 Massen-% mit Bezug auf die Gesamtmenge der kosmetischen Emulsionszusammensetzung beträgt.

4. Kosmetische Emulsionszusammensetzung nach einem der Ansprüche 1 bis 3, wobei Komponente (B) ein hydrophob modifiziertes Polyetherurethan enthält, das die folgende allgemeine Formel (I) aufweist: 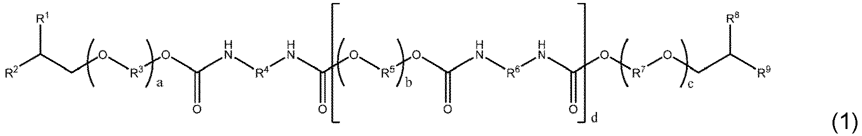 wobei, R¹, R², R⁸ und R⁹ jeweils unabhängig eine C₁₋₂₀)-Kohlenwasserstoffgruppe darstellen; R³, R⁵ und R⁷ jeweils unabhängig eine zweiwertige C₂₋₄-Kohlenwasserstoffgruppe darstellen; R⁴ und R⁶ jeweils unabhängig eine zweiwertige C₃₋₁₆-Kohlenwasserstoffgruppe darstellen; a und c jeweils unabhängig eine Zahl von 10 bis 100 darstellen; b eine Zahl von 100 bis 500 darstellt; und d eine Zahl von 0 bis 10 darstellt.

5. Kosmetische Emulsionszusammensetzung nach einem der Ansprüche 1 bis 4, wobei der Gehalt an Komponente (B) 0,01 bis 5,0 Massen-% in Bezug auf die Gesamtmenge der kosmetischen Emulsionszusammensetzung beträgt.

6. Kosmetische Emulsionszusammensetzung nach einem der Ansprüche 1 bis 5, wobei Komponente (B) 0,01 bis 5 Massenteile pro 1 Masseteil von Komponente (A) in der kosmetischen Emulsionszusammensetzung beträgt.

7. Kosmetische Emulsionszusammensetzung nach einem der Ansprüche 1 bis 6, wobei die kosmetische Emulsionszusammensetzung zusätzlich ein polyethermodifiziertes Tensid von einem Silikon-Typ als eine Komponente (C) enthält.

8. Kosmetische Emulsionszusammensetzung nach einem der Ansprüche 1 bis 7, wobei das Massenverhältnis zwischen der Gesamtmasse der Komponenten, die die wässrige Phase ausmachen, und der Gesamtmasse der Komponenten, die die Ölphase ausmachen, 10 : 90 bis 95 : 5 beträgt (die Gesamtsumme für das Massenverhältnis ist 100).

9. Kosmetische Emulsionszusammensetzung nach einem der Ansprüche 1 bis 8, wobei die Ölphase der kosmetischen Emulsionszusammensetzung ein Silikonöl enthält.

10. Kosmetische Emulsionszusammensetzung nach einem der Ansprüche 1 bis 9, wobei die kosmetische Emulsionszusammensetzung eine Wasser-in-Öl-Emulsion ist.

11. Kosmetische Emulsionszusammensetzung nach einem der Ansprüche 4 bis 10, wobei R¹ und R⁸ jeweils eine Decylgruppe sind; R² und R⁹ jeweils eine Dodecylgruppe sind; R³, R⁵ und R⁷ jeweils eine Ethylengruppe sind; R⁴ und R⁶ jeweils eine Hexylgruppe sind; a und c jeweils 20 sind; und b 240 ist.

12. Verwendung einer Emulsionszusammensetzung, die (A) eine Komponente, die einen Monoalkylglycerylether enthält, der eine C₆₋₈-Alkylgruppe aufweist, und (B) eine Komponente umfasst, die ein assoziatives Urethan für die Herstellung eines Kosmetikums enthält.

13. Verwendung nach Anspruch 12, wobei Komponente (A) einen Cyclohexylglycerylether enthält.

14. Verwendung nach Anspruch 12 oder 13, wobei Komponente (B) ein hydrophob modifiziertes Polyetherurethan enthält, das die folgende allgemeine Formel (I) aufweist: 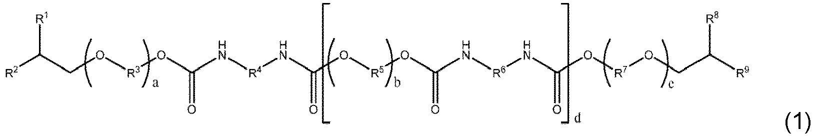 wobei, R¹, R², R⁸ und R⁹ jeweils unabhängig eine C₁₋₂₀)-Kohlenwasserstoffgruppe darstellen; R³, R⁵ und R⁷ jeweils unabhängig eine zweiwertige C₂₋₄-Kohlenwasserstoffgruppe darstellen; R⁴ und R⁶ jeweils unabhängig eine zweiwertige C₃₋₁₆-Kohlenwasserstoffgruppe darstellen; a und c jeweils unabhängig eine Zahl von 10 bis 100 darstellen; b eine Zahl von 100 bis 500 darstellt; und d eine Zahl von 0 bis 10 darstellt.

15. Verwendung nach einem der Ansprüche 12 bis 14, wobei die Emulsionszusammensetzung ferner ein polyethermodifiziertes Tensid von dem Silikon-Typ als eine Komponente (C) umfasst.

## Revendications

1. Composition d'émulsion cosmétique comprenant (A) un composant contenant un monoalkyl glyceryl ether ayant un groupe alkyle en C_{6 à 8} et (B) un composant contenant un uréthane associatif.

2. Composition d'émulsion cosmétique selon la revendication 1, dans laquelle le composant (A) contient un cyclohexyl glyceryl ether.

3. Composition d'émulsion cosmétique selon la revendication 1 ou 2, dans laquelle la teneur en composant (A) est de 0,01 à 10 % en masse par rapport à la quantité totale de la composition d'émulsion cosmétique.

4. Composition d'émulsion cosmétique selon l'une quelconque des revendications 1 à 3, dans laquelle le composant (B) contient un polyéther uréthane hydrophobiquement modifié ayant la formule générale (I) suivante : 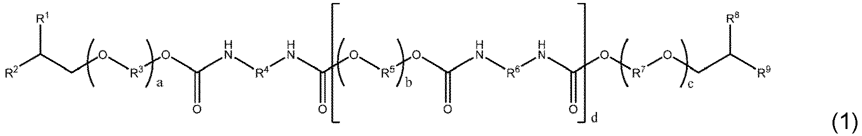 dans laquelle, R¹, R², R⁸ et R⁹ représentent chacun indépendamment un groupe hydrocarbure en C_{1 à 20} ; R³, R⁵ et R⁷ représentent chacun indépendamment un groupe hydrocarbure divalent en C _{2 à 4} ; R⁴ et R⁶ représentent chacun indépendamment un groupe hydrocarbure divalent en C_{3 à 16} ; a et c représentent chacun indépendamment un nombre de 10 à 100 ; b représente un nombre de 100 à 500 ; et d représente un nombre de 0 à 10.

5. Composition d'émulsion cosmétique selon l'une quelconque des revendications 1 à 4, dans laquelle la teneur en composant (B) est de 0,01 à 5,0 % en masse par rapport à la quantité totale de la composition d'émulsion cosmétique.

6. Composition d'émulsion cosmétique selon l'une quelconque des revendications 1 à 5, dans laquelle le composant (B) représente 0,01 à 5 parties en masse pour 1 partie en masse de composant (A) dans la composition d'émulsion cosmétique.

7. Composition d'émulsion cosmétique selon l'une quelconque des revendications 1 à 6, dans laquelle la composition d'émulsion cosmétique contient en outre un tensioactif de type silicone modifié par un polyéther comme composant (C).

8. Composition d'émulsion cosmétique selon l'une quelconque des revendications 1 à 7, dans laquelle le rapport massique entre la masse totale des composants constituant la phase aqueuse et la masse totale des composants constituant la phase huileuse est de 10:90 à 95:5 (le total pour le rapport massique est de 100).

9. Composition d'émulsion cosmétique selon l'une quelconque des revendications 1 à 8, dans laquelle la phase huileuse de la composition d'émulsion cosmétique contient une huile de silicone.

10. Composition d'émulsion cosmétique selon l'une quelconque des revendications 1 à 9, dans laquelle la composition d'émulsion cosmétique est une émulsion eau dans l'huile.

11. Composition d'émulsion cosmétique selon l'une quelconque des revendications 4 à 10, dans laquelle R¹ et R⁸ sont chacun un groupe décyle ; R² et R⁹ sont chacun un groupe dodécyle ; R³, R⁵ et R⁷ sont chacun un groupe éthylène ; R⁴ et R⁶ sont chacun un groupe hexyle ; a et c sont chacun 20 ; et b est égal à 240.

12. Utilisation d'une composition d'émulsion comprenant (A) un composant contenant un monoalkyl glycéryl éther ayant un groupe alkyle en C_{6 à 8} et (B) un composant contenant un uréthane associatif pour la fabrication d'un cosmétique.

13. Utilisation selon la revendication 12, dans laquelle le composant (A) contient un cyclohexyl glyceryl ether.

14. Utilisation selon la revendication 12 ou 13, dans laquelle le composant (B) contient un polyéther uréthane hydrophobiquement modifié ayant la formule générale (I) suivante : 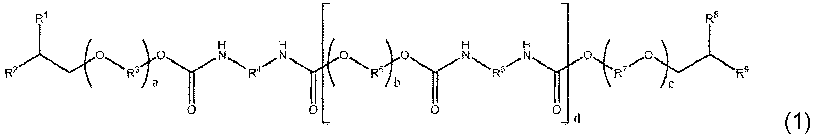 dans laquelle, R¹, R², R⁸ et R⁹ représentent chacun indépendamment un groupe hydrocarbure en C_{1 à 20} ; R³, R⁵ et R⁷ représentent chacun indépendamment un groupe hydrocarbure divalent en C_{2 à 4} ; R⁴ et R⁶ représentent chacun indépendamment un groupe hydrocarbure divalent en C_{3 à 16} ; a et c représentent chacun indépendamment un nombre de 10 à 100 ; b représente un nombre de 100 à 500 ; et d représente un nombre de 0 à 10.

15. Utilisation selon l'une quelconque des revendications 12 à 14, dans laquelle la composition d'émulsion comprend en outre un tensioactif de type silicone modifié par un polyéther comme composant (C).
